(19) Europäisches Patentamt
European Patent Office
Office européen des brevets

(11) Veröffentlichungsnummer: **0 288 764**
**B1**

(12) # EUROPÄISCHE PATENTSCHRIFT

(45) Veröffentlichungstag der Patentschrift: **24.10.90**

(51) Int. Cl.$^5$: **C07C 221/00**, C07D 207/08, C07C 213/00, C07C 215/04, C07C 255/32, C07C 229/40

(21) Anmeldenummer: **88105044.7**

(22) Anmeldetag: **29.03.88**

(54) **Optisch aktive alpha-Aminoaldehyde, Verfahren zu ihrer Herstellung und ihre Verwendung zur stereoselektiven Herstellung optisch aktiver beta-Aminoalkohole.**

(30) Priorität: **08.04.87 DE 3711911**

(43) Veröffentlichungstag der Anmeldung: **02.11.88 Patentblatt 88/44**

(45) Bekanntmachung des Hinweises auf die Patenterteilung: **24.10.90 Patentblatt 90/43**

(84) Benannte Vertragsstaaten: **CH DE FR GB IT LI**

(56) Entgegenhaltungen:
**EP-A- 0 100 717**

**ANGEWANDTE CHEMIE, Bd 99, Nr. 11, 1987; Weinheim M.T. REETZ et al. "Stereoselektive Synthese von beta-Aminoalkoholen aus opt. aktiven alpha-Aminosäuren" Seiten 1186-1187**
**PATENT ABSTRACTS OF JAPAN, unexamined applications, Field C, Band 7, Nr. 268, 30. Nov. 1983; THE PATENT OFFICE JAPANESE GOVERNMENT Seite 144 C 197**
**CHEMICAL ABSTRACTS, Band 79, Nr. 23, 10. Dez. 1973, Columbus, Ohio, USA; L. DUHAMEL "Synthesis of alpha-aminoaldehydes by reduction of alpha-aminoamides and alpha-amino esters" Seite 364, Zusammenfassung-Nr. 136 951z**
**JOURNAL OF THE AMERICAN CHEMICAL SOCIETY,**

(73) Patentinhaber: **BAYER AG, D-5090 Leverkusen 1 Bayerwerk(DE)**

(72) Erfinder: **Reetz, Manfred T., Prof. Dr., Wiesentalweg 14, D-3550 Marburg(DE)**
Erfinder: **Drewes, Mark W., Friedrich-Ebert-Strasse 111, D-3550 Marburg(DE)**

(56) Entgegenhaltungen: (Fortsetzung)
**Band 109, No. 1, 7. Jan. 1987; W.D. LUBELL et al. "Configurational stability of N-protected alpha-aminoaldehydes" Seiten 236-239**
**CHEMICAL ABSTRACTS, Band 73, Nr. 15, 12. Okt. 1970, Columbus, Ohio, USA; L.DUHAMEL "Rearrangement of alpha-aminoaldehydes to alpha-amino ketones. Influence of steric factors: application of the Newman rule to 1-ene-1,2-diamine intermediates having a neopentyl group" Seite 311, Zusammenfassung-Nr. 76575u"die Sicherheitstechnik beim Umgang mit Braun 000**
**CHEMICAL ABSTRACTS, Band 77, Nr. 15, 9. Okt. 1972, Columbus, Ohio, USA; P. CUHAMEL et al. "Addition to carbonyls of alpha-(N,N-dialkylamino)aldehydes and ketones. Asymmetric induction and effect of nitrogen substituents" Seite 330, Zusammenfassung-Nr. 100 337ygroup" Seite 311, Zusammenfassung-Nr. 76575u"die Sicherheitstechnik beim Umgang mit Braun 000**
**ANGEWANDTE CHEMIE, Bd 96, Nr. 8, 1984, Weinheim; M.T. REETZ "Chelat-oder Nicht-chelat-Kontrolle bei Additionsreaktionen von chiralen alpha-und beta-Alkoxycarbonyl-Verbindungen" Seiten 542-555**

**Die Akte enthält technische Angaben, die nach dem Eingang der Anmeldung eingereicht wurden und die nicht in dieser Patentschrift enthalten sind.**

EP 0 288 764 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft neue, optisch aktive α-Aminoaldehyde mit geschützter Aminogruppe, Verfahren zu ihrer Herstellung und ihre Verwendung zur stereoselektiven Herstellung von optisch aktiven β-Aminoalkoholen.

Optisch aktive α-Aminoaldehyde mit geschützter Aminogruppe sind wichtige Zwischenprodukte für die Herstellung optisch aktiver β-Aminoalkohole. Die optisch aktiven β-Aminoalkohole sind ihrerseits wichtige Zwischenprodukte für die Herstellung biologisch aktiver Verbindungen und zwar sowohl natürlich vorkommender als auch synthetisch hergestellter Wirkstoffe. Bei diesen Wirkstoffen handelt es sich vielfach um Di- oder Oligopeptide, die optisch aktive β-Aminoalkohole als Kettenglieder enthalten, zum Beispiel Renin-Inhibitoren mit blutdrucksenkender Wirkung (siehe CA 104, (1986) 19 822 g und Tetrahedron Letters, Vol. 27, No. 21, S. 2337-2340, (1986)); Diät-Süßstoffen (siehe EP-A 1-0068 551) und die Antitumor-Wirksamkeit des Bleomycins verstärkenden Bestatin-Derivaten (siehe DE-OS 2 632 396).

Wegen der großen Bedeutung der optisch aktiven α-Aminoaldehyde als Zwischenprodukte für die Herstellung von optisch aktiven β-Aminoalkoholen aus natürlich vorkommenden Aminosäuren (= optisch aktiven α-Aminosäuren) sind die Herstellung der optisch aktiven α-Aminoaldehyde und deren weitere Umsetzung zu den optisch aktiven Aminoalkoholen intensiv bearbeitet worden. Von besonderer Bedeutung bei dieser angegebenen Reaktionsfolge ist, daß sämtliche Reaktionsschritte, d.h. sowohl die Reduktion der optisch aktiven Aminosäuren zu den α-Aminoaldehyden als auch die Umsetzung der α-Aminoaldehyde zu den β-Aminoalkoholen unter Erhalt der Konfiguration am α-C-Atom und stereoselektiv bei der Ausbildung des neuen optisch aktiven β-C-Atoms verlaufen. Da die Konfigurationsstabilität der α-Aminoaldehyde und die Steroselektivität ihrer Umsetzungen mit metallorganischen Verbindungen und Enolaten zu den entsprechenden β-Aminoalkoholen wesentlich durch die am Stickstoff des α-Aminoaldehyds befindliche Schutzgruppe bestimmt werden, wurden die verschiedensten Schutzgruppen zur Maskierung der Aminogruppe der α-Aminoaldehyde angewendet. Aber die mit diesen bislang verwendeten Schutzgruppen erreichten Konfigurationsstabilitäten und Stereoselektivitäten sind unzureichend, wie die bei der Umsetzung der N-geschützten, optisch aktiven α-Aminoaldehyde erhaltenen Gemische der diastereomeren β-Aminoalkohole und die optische Reinheit der einzelnen diastereomeren β-Aminalkohole zeigen.

Im einzelnen wurden bislang folgende Schutzgruppen zur Maskierung der Aminogruppe der optisch aktiven α-Aminosäuren und der aus ihnen hergestellten optisch aktiven α-Aminoaldehyde verwendet:

1. Die Tertiärbutoxycarbonylgruppe (Boc):

Die α-Aminoaldehyde mit einer durch eine Boc-Gruppe geschützten Aminogruppe haben den Nachteil, daß sie nicht immer konfigurationsstabil sind und bei der Umsetzung mit metallorganischen Verbindungen und Enolaten Gemische der diastereomeren β-Aminoalkohole liefern (siehe die entsprechenden Angaben in Tetrahedron Letters loc. cit. und J. Am. Chem. Soc. 1987, 109, S. 236-239).

2. Die Triphenylmethylgruppe (Tritylgruppe):

Die am Stickstoff durch eine Tritylgruppe geschützten α-Aminoaldehyde weisen nur eine unzureichende Konfigurationsstabilität auf und liefern bei ihrer Umsetzung mit metallorganischen Verbindungen und Enolaten Gemische der beiden diastereomeren Alkohole (siehe die Angaben in Tetrahedron Letters loc. cit.).

3. Die 9-(9-Phenylfluorenyl)-Gruppe:

Die am Stickstoff durch die 9-(9-Phenylfluorenyl)-Gruppe geschützten α-Aminoaldehyde weisen zwar eine verbesserte Konfigurationsstabilität auf, bei der Umsetzung mit metallorganischen Verbindungen oder Enolaten bilden sich jedoch Gemische der beiden diastereoisomeren β-Aminoalkohole (s. J. Am. Chem. Soc. 1987, 109, S. 236-239).

Es wurde nun gefunden, daß α-Aminoaldehyde mit einer durch Alkyl-, Alkenyl-, Cycloalkyl- oder Aralkyl-Gruppen vollständig geschützten d.h. peralkylierten Aminogruppe eine überraschend hohe Konfigurationsstabilität aufweisen und mit metallorganischen Verbindungen und Enolaten stereoselektiv zu den entsprechenden ß-Aminoalkoholen reagieren.

Die Erfindung betrifft daher optisch aktive, am Stickstoff substituierte α-Aminoaldehyde der Formeln

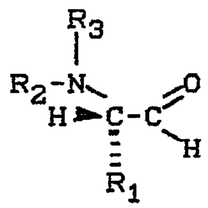 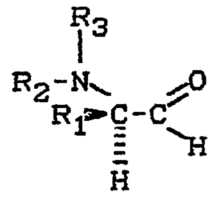 I

S-Form R-Form

in denen
$R_1$ für einen gegebenenfalls substituierten Alkyl-, Alkenyl-, Aralkyl- oder Aryl-Rest steht und
$R_2$ und $R_3$ unabhängig voneinander eine gegebenenfalls substituierte Benzylgruppe bedeuten, zusammen eine gegebenenfalls substituierten Phenylen-(1,2)-bismethylen-Rest bilden oder $R_2$ ein gegebenenfalls substituierter Benzyl-Rest ist und $R_3$ zusammen mit $R_1$ einen Propylen-1, 3-Rest bildet.

Die α-Aminoaldehyde der Formel I werden im Rahmen der vorliegenden Erfindung vereinfachend als peralkylierte Aminoaldehyde bezeichnet. Weil die Benzylgruppen besonders einfach abspaltbar sind, sind die perbenzylierten Aminoaldehyde der Formel I bevorzugt.

Für $R_1$ seien als gegebenenfalls substituierte Alkylreste beispielsweise genannt: $C_1$-$C_{12}$-Alkylreste wie der Methyl-, Ethyl-, iso-Propyl-, Butyl-, sec.-Butyl-, iso-Butyl-, Amyl- und Hexyl-Rest; als Substituenten kommen für die Alkylreste vorzugsweise die Amino-, Hydroxy-, Benzyloxy-, Benzylmercapto-, Carbonamido- oder eine gegebenenfalls am N substituierte Imidazolyl-4- oder Indolyl-3-Gruppe in Betracht. Als gegebenenfalls substituierte Alkylreste seien beispielsweise genannt: der Benzyloxymethyl-, Benzylmercaptomethyl-, 1-Benzyloxyethyl-, 2-Methylmercaptoethyl-, Carbamoylmethyl-, 2-Carbamoylethyl-, 3-(N,N-Dibenzylamino)-propyl-, 4-(N,N-Dibenzylamino)-butyl-, der (Imidazolyl-4)-methyl-, und der (Indolyl-3)-methyl-Rest.

Als gegebenenfalls substituierte Alkenylreste sei beispielsweise der Vinyl-Rest genannt.

Als gegebenenfalls substituierte Aralkylreste seien beispielsweise genannt: der Benzylrest und durch $C_1$-$C_4$-Alkyl, $C_1$-$C_4$-Alkoxy, Benzyloxy oder Halogenatome substituierte Benzylreste, wie der 2- und 4-Methylbenzylrest, der 4-Methoxybenzyl- und der 4-Benzyloxybenzyl-Rest.

Als gegebenenfalls substituierte Arylreste seien beispielsweise genannt der Phenylrest und durch OH–, $C_1$–$C_4$ Alkoxy- und $C_1$–$C_4$-Alkylgruppen substituierte Phenylreste wie der 4-Hydroxyphenylrest, der 4-Methoxyphenylrest und der 2-Methyl- und 4-Methyl-phenylrest.

Für $R_2$ und $R_3$ seien als gegebenenfalls substituierte Benzylreste die durch $C_1$–$C_4$-Alkylgruppen, $C_1$-$C_4$-Alkoxy- und Benzyloxygruppen oder durch Halogenatome substituierten Benzyl- und Phenylen-(1, 2)-bis-methylen-Reste genannt; zum Beispiel der 2- und 4-Methylbenzylrest, der 2- und 4-Methoxybenzylrest und der α-Methylbenzylrest.

Die Erfindung betrifft ferner ein Verfahren zur Herstellung der optisch aktiven, am N peralkylierten Aminoaldehyde der Formel I; das Verfahren ist dadurch gekennzeichnet, daß man optisch aktive, am Stickstoff peralkylierte Aminosäuren oder deren Ester der Formeln

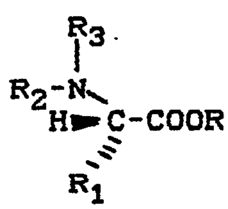 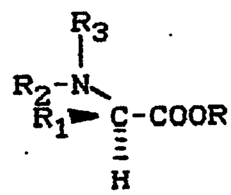 II

S-Form R-Form

in denen
$R_1$, $R_2$, $R_3$ die unter Formel I angegebene Bedeutung haben und
R für Wasserstoff, einen Alkyl- oder Aralkyl-Rest steht,
nach an sich bekannten Verfahren zu den am Stickstoff peralkylierten α-Aminoaldehyden der Formel I reduziert.

Bekannte Verfahren zur Reduktion von am N geschützten α-Aminocarbonsäuren zu am N geschützten α-Aminoaldehyden sind zum Beispiel:

a) Die Reduktion der α-Aminocarbonsäuren oder deren Ester mit Lithiumaluminiumhydrid und Oxidation der so erhaltenen am Stickstoff geschützten β-Aminoalkohole mit Dimethylsulfoxid/Oxalylchlorid oder Chromtrioxid/Pyridin zu den am N geschützten α-Aminoaldehyden;
b) die Reduktion der Aminocarbonsäuren oder deren Ester mit Diisobutylaluminiumhydrid zu den am N geschützten α-Aminoaldehyden.

Am N peralkylierte α-Aminosäuren und ihre Herstellung sind bekannt (siehe Beilstein E IV, Bd. 12 S. 2287, 2295–2297; System-Nr, 1699). Die am N peralkylierten, optisch aktiven α-Aminosäuren werden analog durch Alkylieren entsprechender optisch aktiver α-Aminosäuren, zum Beispiel natürlich vorkommender Aminosäuren, hergestellt.

Eine besonders vorteilhafte Ausführungsform des erfindungsgemässen Herstellungsverfahrens für die Aminoaldehyde der Formel I besteht darin, die optisch aktiven α-Aminosäuren durch Umsetzung mit Benzylhalogeniden, wie Benzylchlorid oder – besonders bevorzugt – mit Benzylbromid, in einem Arbeitsgang am Stickstoff zu alkylieren und die Carboxylgruppe in eine Carbonsäureestergruppe zu überführen.

Als optisch aktive α-Aminosäuren werden bevorzugt die in der Natur vorkommenden optisch aktiven α-Aminosäuren verwendet.

Die auf diese Weise erhaltenen, am N peralkylierten Aminosäureester, werden anschliessend, wie vorstehend angegeben, in bekannter Weise entweder durch Reduktion mit Lithiumaluminiumhydrid und Oxidation mit Dimethylsulfoxid/Oxalylchlorid oder durch Reduktion mit Diisobutylaluminiumhydrid in die erfindungsgemäßen peralkylierten Aminoaldehyde der Formel I überführt.

Die Erfindung betrifft ferner die Verwendung der erfindungsgemäßen optisch aktiven, am N peralkylierten α-Aminoaldehyde der Formel I zur Herstellung von optisch aktiven β-Aminoalkoholen der Formeln

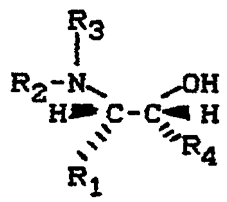

A

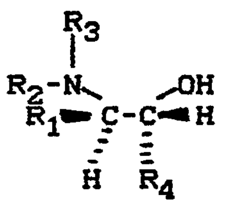

B

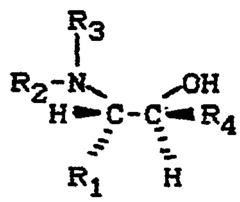

C

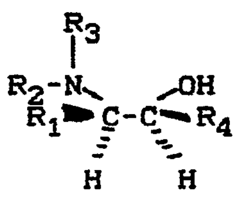

D

III

in denen

$R_1$, $R_2$ und $R_3$ die unter Formel I angegebene Bedeutung haben und

$R_4$ für einen gegebenenfalls substituierte Alkyl-, Alkenyl-, Alkinyl-, Aralkyl-, Aryl- oder Heteroaryl-Rest oder die CN-Gruppe steht.

Als Substituenten für die Alkyl-, Alkenyl-, Alkinyl-, Aralkyl-, Phenyl- und Heteroaryl-Reste seien beispielsweise geschützte Hydroxy-, Mercapto- und Aminogruppen, ferner Sulfoxid-, Sulfon-, Nitro- und Cyan-Gruppen, genannt.

Zur Herstellung der optisch aktiven β-Aminoalkohole der Formel III werden die erfindungsgemäßen, optisch aktiven α-Aminoaldehyde der Formel I in an sich bekannter Weise (siehe z.B. Angew. Chem. 96, S. 542-555, (1984)) mit metallorganischen Verbindungen oder Enolaten in einem unter den Reaktionsbedingungen inerten organischen Lösungsmittel umgesetzt.

Als metallorganische Verbindungen seien beispielsweise genannt:

Metallorganische Verbindungen, bei denen das Metallatom nur mit organischen Gruppen verbunden ist (sog. einheitliche m.V.) wie Alkyl-, Vinyl-, Alkinyl-, Aryl- oder Heteroaryl-Lithium, z.B. Methyl-, Methoxymethyl-, Cyanomethyl-, Phenylsulfonyl-methyl-, N,N-Dialkylaminomethyl-, Butyl-, Allyl-, 3-Alkoxyallyl-, Vinyl-, 1-Ethoxyvinyl- und Phenyl-Lithium; ferner 2-Li-furan, 2-Li-4-methyl-1,3-thiazol, 5-Li-1-methyl-pyrazol, 2-Li-pyridin und α-Picolyl-Li; Nitromethyl-Natrium; Dialkyl- oder Dialkenyl-Zink, z.B. Dimethyl- oder Diallyl-Zink; Dialkylmagnesium z.B. Dimethylmagnesium und Silane wie Allyltrimethylsilan.

Metallorganische Verbindungen, bei denen das Metallatom außer mit organischen Gruppen auch noch mit anderen Gruppen, Atomen oder Ionen verbunden ist (sog. gemischte m.V.) z.B. Grignard- und Grignard-analoge Verbindungen wie Alkyl-, Alkenyl-, Alkinyl-, Aralkyl- oder Aryl-Magnesium-halogenide, wie Methyl-magnesium-chlorid oder -iodid, Allyl-magnesium-bromid, Benzyl-magnesium-iodid, Phenyl-magnesium-bromid, Alkyl-, Alkenyl-, Alkinyl-, Aralkyl- oder Aryl-Mangan-halogenide wie $CH_3MnBr$, Alkyl-, Alkenyl-, Alkinyl-, Aralkyl- oder Aryl-titan-chloride oder -alkoxylate wie $CH_3TiCl_3$ oder $CH_3Ti(OC_3H_7i)_3$, ferner gemischte Silane wie $(CH_3)_3SiCN$.

Einige der metallorganischen Verbindungen, z.B. Dimethyl- oder Dibutylzink, ferner Silane wie Allyltrimethylsilan, werden in Kombination mit Fluoridionen oder Lewis-Säuren angewendet z.B. $(CH_3)_2Zn/TiCl_4$, Allyltrimethylsilan/$SnCl_4$, $(CH_3)_3SiCN/F^-(NR'_4)^+$, $(CH_3)_3SiCN/BF_3$, $(CH_3)_3SiCN/MgBr_2$.

Als Enolate seien beispielsweise genannt: Lithium-, Bor- und Titan-Enolate von gegebenenfalls substituierten Carbonsäureestern (z.B. Essigester, Benzyloxylessigsäureethylester, α-Isocyanoessigsäureethylester oder N,N-Dibenzylaminoessigsäureethylester) und Ketonen, ferner die O-Trimethylsilylketenketale dieser Carbonsäureester und O-Trimethylsilylenolsilane der Ketone. Unter Enolaten werden im Rahmen der vorliegenden Erfindung auch Enolat-analoge Verbindungen wie α-lithiierte Imine oder lithiierte Bis-lactimether verstanden.

O-Trimethylsilylketenketale und O-Trimethylsilylenolsilane werden in Kombination mit Lewis-Säuren wie $BF_3$, $MgBr_2$ oder $SnCl_4$ angewendet.

Diese Additionsreaktionen verlaufen mit hoher Stereoselektivität. Die erhaltenen β-Aminoalkohole der Formel III bestehen im Durchschnitt zu 90 bis 97 % aus einem einzigen Diastereomer und enthalten nur noch etwa 10 bis 3 % des anderen Diastereomeren.

Die optische Reinheit der bei der Umsetzung der erfindungsgemäßen α-Aminoaldehyde der Formel I mit metallorganischen Verbindungen und Enolaten erhaltenen β-Aminoalkohole der Formel III wurde durch $^{13}C$-NMR-spektros kopische Untersuchung der durch O-Acylieren der Aminoalkohole mit (+)- und (-)-α-Methoxy-α-(trifluormethyl)phenylessigsäurechlorid erhaltenen Ester bestimmt. Danach beträgt die optische Reinheit der einzelnen Diastereomere mindestens 98 %, d.h. weder bei der Darstellung noch bei den Reaktionen der Aldehyde I tritt eine nennenswerte Racemisierung am α-C-Atom ein.

Es wurde gefunden, daß die Umsetzung der erfindungsgemäßen peralkylierten Aminoaldehyde der Formel I mit metallorganischen Verbindungen und Enolaten stereoselektiv unter Bildung der nicht chelatkontrollierten diastereomeren β-Aminoalkohole der Formeln IIIA oder IIID erfolgt, je nachdem, ob die S- oder die R-Form der α-Aminoaldehyde der Formel I eingesetzt wurde, während die Umsetzung mit metallorganischen Verbindungen in Kombination mit Lewis-Säuren wie $TiCl_4$ oder $SnCl_4$ zur Bildung der chelatkontrollierten diastereomeren β-Aminoalkohole der Formeln IIIB bzw. IIIC führt. Nur die Umsetzung von $(CH_3)_3SiCN$ in Kombination mit Zinkhalogeniden oder $BF_3$ führt zur Bildung diastereomerer β-Aminoalkohole der Formeln III A bzw. III D.

Die Umsetzung der erfindungsgemäßen α-Aminoaldehyde der Formel I mit metallorganischen Verbindungen kann in an sich bekannter Weise vorgenommen werden (siehe z.B. die Angaben in J. Am. Chem. Soc., Vol. 109, No. 1, 1987, S. 237-238).

Die N,N-disubstituierten β-Aminoalkohole der Formel III eignen sich als Katalysatoren für die in Chem. Comm. (ICCCAT) (6) S. 467/468 (1987) beschriebene enantioselektive Addition von Diethylzink an Aldehyde.

Aus den β-Aminoalkoholen der Formel III lassen sich die Benzylgruppen in an sich bekannter Weise durch katalytische Hydrierung (Hydrogenolyse) abspalten. Die Hydrogenolyse wird vorzugsweise mit Pd-Schwarz/Kohle unter Verwendung von Ameisensäure als Wasserstoffdonator vorgenommen. Die bei der Hydrogenolyse zunächst gebildeten Formamide werden anschließend entweder alkalisch durch Zugabe von Basen oder aber - bevorzugt - sauer durch Zugabe verdünnter Salzsäure in Gegenwart von Kieselgel hydrolysiert.

<u>Beispiel 1</u>

a)

10 g (= 42 mmol) Alanin, 9,0 g (= 224 mmol) NaOH und 46,4 g (= 336 mmol) $K_2CO_3$ werden in 100 ml 5 %igem Ethanol gelöst. Die Lösung wird 10 Minuten auf Rückflußtemperatur erwärmt und dann mit 61,3 g (= 358 mmol) Benzylbromid versetzt. Das Reaktionsgemisch wird 1 Stunde auf Rückflußtemperatur erhitzt. Anschließend wird das Reaktionsgemisch abgekühlt und das Ethanol im Vakuum abdestilliert. Die zurückbleibende wäßrige Lösung wird mit Ether extrahiert. Die vereinigten Etherextrakte werden mit gesättigter Kochsalzlösung gewaschen, über $MgSO_4$ getrocknet und im Vakuum vom Ether befreit. Der Rückstand wird durch Chromatographie an einer Kieselgel-Säule gereinigt (PE/EtOAc; 98:2). Es werden 27,8 g (= 69 % der Theorie) S-α-(N,N-Dibenzylamino)-propionsäurebenzylester in Form eines farblosen Öls erhalten.

Bei gleicher Arbeitsweise wurden bei Verwendung von 42 mmol Phenylalanin, 42 mmol Prolin, 42 mmol Leucin oder 42 mmol Valin anstelle der 42 mmol Alanin in vergleichbaren Ausbeuten und ebenfalls in Form farbloser Öle erhalten:

a1) S-α-(N,N-Dibenzylamino)-β-phenylpropionsäurebenzylester,
a2) S-(N-Benzylamino)-pyrrolidin-(2)-carbonsäurebenzylester,
a3) S-α-(N,N-Dibenzylamino)-β-4-methylpentancarbonsäurebenzylester bzw.
a4) S-α-(N,N-Dibenzylamino)-3-methylbutancarbonsäurebenzylester.

b)

α)Die Suspension von 2,56 g (= 67 mmol) Lithiumaluminiumhydrid in 75 ml Ether wird unter Rühren und Kühlen bei 0°C tropfenweise mit einer Lösung von 20,2 g (= 56 mmol) S-α-(N,N-Dibenzylamino)-propionsäurebenzylester in 20 ml Ether versetzt. Nach beendeter Zugabe wird das Kühlbad entfernt und das Reaktionsgemisch 2 Stunden bei Raumtemperatur gerührt. Anschließend wird das Reaktionsgemisch durch das Nacheinanderzugeben von 3 ml $H_2O$, 3 ml 15 %iger Natronlauge und 9 ml $H_2O$ hydrolysiert. Die entstandenen Hydroxide werden abfiltriert und zweimal mit Ether ausgekocht. Das wäßrige Filtrat wird nach Abtrennen der organischen Schicht mehrfach mit Ether extrahiert. Die vereinigten Extrakte werden über Magnesiumsulfat getrocknet und anschließend im Vakuum eingeengt. Der Rückstand wird durch Chromatographie an einer Kieselgel-Säule gereinigt (Petrolether/Ethylacetat; 95:5). Es werden 10,4 g (= 73 % der Theorie) S-2-(N,N-Dibenzylamino)-propanol in Form weißer Kristalle (Fp. 39°C) erhalten.

Auf die gleiche Weise wurden durch Reduktion des S-α-(N,N-Dibenzylamino)-β-phenyl-propionsäurebenzylesters, des S-(N-Benzylamino)-pyrrolidin-(2)-carbonsäurebenzylesters, des S-α-(N,N-Dibenzylamino)-4-methylpentancarbonsäurebenzylesters und des S-α-(N,N-Dibenzylamino)-3-methylbutancarbonsäurebenzylesters S-2-(N,N-Dibenzylamino)-3-phenyl-propanol, S-(N-Benzylamino)-(2)-hydroxymethylpyrrolidin, S-2-(N,N-Dibenzylamino)-4-methylpentanol-1 bzw. S-2-(N,N-Dibenzylamino)-3-methylbutanol-1 erhalten.

β)Die auf -60°C abgekühlte Lösung von 270 mg (= 2,11 mmol) Oxalylchlorid in 10 ml Methylenchlorid wird tropfenweise mit 301 mg (= 3,92 mmol) Dimethylsulfoxid versetzt. Die Lösung wird 5 Minuten gerührt und anschließend tropfenweise mit 500 mg (= 2 mmol) S-2-(N,N-Dibenzylamino)-propanol versetzt. Das Reaktionsgemisch wird 30 Minuten bei -60°C gerührt und anschließend mit 0,99 g (= 9,8 mmol) Triethylamin versetzt. Nach 15-minütigem Rühren wird das Reaktionsgemisch auf Raumtemperatur erwärmt. Die Methylenchloridlösung wird mit 1,5 %iger Salzsäure und anschließend mit 5 %iger Sodalösung gewaschen, über Magnesiumsulfat getrocknet und anschließend im Vakuum vom Methylenchlorid befreit.

Es werden 0,44 g (= 88 % der Theorie) S-α-(N,N-Dibenzylamino)-propionaldehyd in Form eines gelben Öls erhalten. Die Verbindung kann ohne weitere Reinigung für die nachstehend beschriebenen Additionsreaktionen verwendet werden.

In gleicher Weise wurden auch S-2-(N,N-Dibenzylamino)-3-phenyl-propanol-1, S-2-(Hydroxymethyl)-(N-benzylamino)-pyrrolidin und S-2-(N,N-Dibenzylamino)-4-methylpentanol-1 und S-2-(N,N-Dibenzylamino)-3-methylbutanol-1 zum S-2-(N,N-Dibenzylamino)-β-phenylpropionaldehyd, zum S-(N-Benzylamino)-pyrrolidin-(2)-aldehyd, zum S-2-(N,N-Dibenzylamino)-4-methylpentanal bzw. zum S-2-(N,N-Dibenzylamino)-3-methylbutanal oxidiert.

c)

α)Die Lösung von 1 g (= 4 mmol) S-α-(N,N-Dibenzylamino)-propionaldehyd in 3 ml Ether wird zu einer Lösung von 4,8 mmol Methylmagnesiumiodid in 4 ml Ether bei 0°C getropft. Nach 1-stündigem Rühren wird das Reaktionsgemisch durch Zugabe von 8 ml gesättigter Ammoniumchloridlösung hydrolysiert, die organische Phase abgetrennt und die wäßrige Phase mit Ether extrahiert. Die vereinigten etherischen Phasen werden mit Kochsalzlösung gewaschen und über Magnesiumsulfat getrocknet. Der nach Abdestillieren des Ethers zurückbleibende Rückstand wird durch Chromatographie gereinigt. Es werden 925 mg (= 87 % der Theorie) (2R,3S)-3-(N,N-Dibenzylamino)-propanol-(2) in Form weißer Kristalle vom Schmelzpunkt 76 - 77°C erhalten.

β)Die Lösung von 1,2 mmol Dimethylzink in 10 ml trockenem Methylenchlorid wird bei -50°C mit 2,37 mmol Titantetrachlorid verrührt. Nach 45-minütigem Rühren wird die Lösung auf -78°C abgekühlt und mit 2,37 mmol S-α-(N,N-Dibenzylamino)-propionaldehyd versetzt. Man läßt das Reaktionsgemisch auf -40°C kommen, rührt 3 Stunden bei dieser Temperatur und gießt es anschließend auf Wasser. Die organische Phase wird abgetrennt und die wäßrige Phase mit Methylenchlorid extrahiert. Die vereinigten Methylenchloridphasen werden mit gesättigter Natriumbicarbonat-Lösung gewaschen und über Magnesiumsulfat getrocknet. Nach dem Abdestillieren des Methylenchlorids werden 523 mg (= 82 % der Theorie) (2S,3S)-3-(N,N-Dibenzylamino)-propanol-(2) in Form eines analysenreinen Öls gewonnen. Die $^{13}C$-NMR-spektroskopische Bestimmung des Diastereomerenverhältnisses ergab ein Diastereomerenverhältnis von 94:6.

γ)Die Lösung von 4 mmol Phenylmagnesiumbromid in 5 ml Ether wird bei 0°C mit der Lösung von 4 mmol S-α-(N,N-Dibenzylamino)-propionaldehyd in 5 ml Ether versetzt. Nach 2-stündigem Rühren wird das Reaktionsgemisch mit gesättigter Ammoniumchloridlösung versetzt. Nach dem Abtrennen der organischen Phase wird die wäßrige Phase mit Ether extrahiert. Die vereinigten etherischen Phasen werden mit Kochsalzlösung gewaschen. Nach dem Trocknen über Magnesiumsulfat und Entfernen des Lösungsmittels werden 1,11 g (= 85 % der Theorie) (1R,2S)-1-Phenyl-2-(N,N-Dibenzylamino)-propanol-(1) in Form eines gelben analysenreinen Öls erhalten.

Dieses Öl ist identisch mit N,N-Dibenzylaminonorephedrin.

Zum Entfernen der Benzylgruppe werden 3,9 mmol des (1R,2S)-1-Phenyl-2-(N,N-Dibenzylamino)-propanols-(1) in 5 ml Methanol gelöst. Diese Lösung gibt man zu einer Mischung von 40 ml Methanol, 0,6 g Ameisensäure und 50 mg Palladium-schwarz. Die Luft wird aus dem Reaktionsgefäß mit Wasserstoff verdrängt. Nach 1-stündigem Hydrieren wird der Palladium-Katalysator abfiltriert und die Lösung im Vakuum eingeengt. Der Rückstand wird in 10 ml Methanol aufgenommen und mit 1 g Kieselgel und 5 Tropfen konzentrierter Salzsäure 1 Stunde bei Raumtemperatur gerührt. Der nach dem Abfiltrieren des Kieselgels und Einengen des Filtrates anfallende Rückstand wird durch Chromatographie gereinigt. Es werden 243 mg (= 60 % der Theorie) analysenreines Norephedrin erhalten.

δ)Die Lösung von 2 mmol Lithiumdiisopropylamid in 25 ml Tetrahydrofuran wird bei -78°C tropfenweise mit 2 mmol Essigsäuremethylester versetzt. Nach 15-minütigem Rühren wird die so erhaltene Enolat-Lösung mit der Lösung von 2 mmol S-α-(N,N-Dibenzylamino)-propionaldehyd in 3 ml Tetrahydrofuran versetzt. Das Reaktionsgemisch wird 15 Minuten gerührt und an schließend in gesättigte Natriumbicarbonat-Lösung gegeben. Die organische Phase wird abgetrennt und die wäßrige Phase mehrmals mit Ether extrahiert. Die vereinigten organischen Phasen werden gewaschen und über Magnesiumsulfat getrocknet. Nach dem Abdestillieren des Lösungsmittels werden 529 mg (= 82 % der Theorie) (3R,4S)-3-Hydroxy-4-(N,N-dibenzylamino)-pentansäuremethylester in Form eines analysenreinen Öls erhalten.

In der nachstehenden Tabelle 1 sind die erfindungsgemäß hergestellten β-Aminoalkohole aufgeführt, die bei der Addition der in der Tabelle 1 angegebenen Agentien an S-α-(N,N-Dibenzylamino)-propionaldehyd erhalten wurden. In der Tabelle 1 sind ferner die Ausbeuten und das Diastereomeren-Verhältnis angegeben, in denen die betreffenden β-Aminoalkohole erhalten wurden.

In Tabelle 2 sind die β-Aminoalkohole aufgeführt, die bei der Addition der in Tabelle 2 angegebenen nukleophilen Agentien an S-α-(N,N-Dibenzylamino)-β-phenyl-propionaldehyd erhalten wurden.

In Tabelle 3 sind die β-Aminoalkohole aufgeführt, die bei der Addition der in Tabelle 3 angegebenen nukleophilen Agentien an S-α-(N,N-Dibenzylamino)-β-isopropyl-propionaldehyd erhalten wurden.

In Tabelle 4 sind die β-Aminoalkohole aufgeführt, die bei der Addition der in der Tabelle 4 angegebenen nukleophilen Agentien an S-α-(N,N-Dibenzylamino)-4-methylpentanal erhalten wurden.

Tabelle 1

| Agens | Ausbeute [% d.Th.] | $R_4$ | 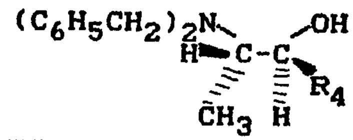 | + | 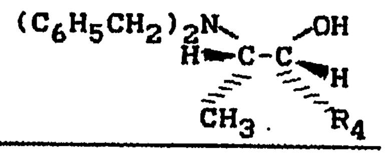 |
|---|---|---|---|---|---|
| $CH_3MgJ$ | 87 | $-CH_3$ | 5 | : | 95 |
| $CH_3Li$ | 91 | $-CH_3$ | 9 | : | 91 |
| $CH_3Ti(OC_3H_7i)_3$ | 78 | $-CH_3$ | 3 | : | 97 |
| $C_6H_5MgBr$ | 85 | $-C_6H_5$ | 3 | : | 97 |
| $C_2H_5MgBr$ | 85 | $-C_2H_5$ | 5 | : | 95 |
| $i-C_3H_7MgBr$ | 75 | $-iC_3H_7$ | <4 | : | >96 |
| tert. $C_4H_9Li$ | 88 | -tert. $C_4H_9$ | <4 | : | >96 |
| $(CH_3)_2Zn/TiCl_4$ | 82 | $-CH_3$ | 94 | : | 6 |
| 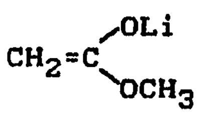 | 82 | $-CH_2COOCH_3$ | 5 | : | 95 |
| 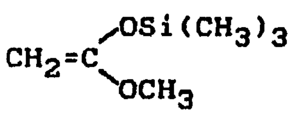 $/BF_3$ | 59 | $-CH_2COOCH_3$ | 4 | : | 96 |

**Tabelle 1** (Fortsetzung)

| Agens | Ausbeute [% d.Th.] | $R_4$ | 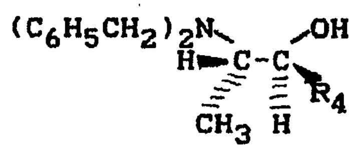 | + | 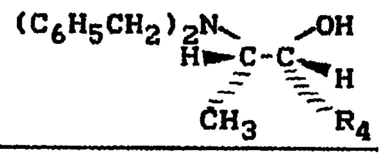 |
|---|---|---|---|---|---|
| 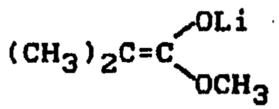 | 84 | $-C(CH_3)_2COOCH_3$ | 3 | : | 97 |
| 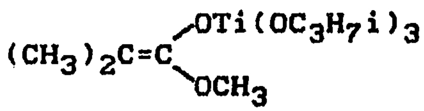 | 72 | $-C(CH_3)_2COOCH_3$ | 4 | : | 96 |
| 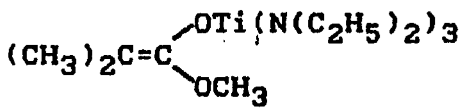 | 73 | $-C(CH_3)_2COOCH_3$ | 3 | : | 97 |

Tabelle 2

| Agens | Ausbeute [% d.Th.] | $R_4$ | 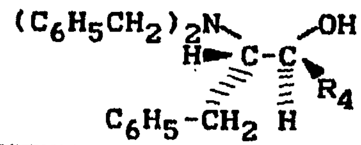 | + | 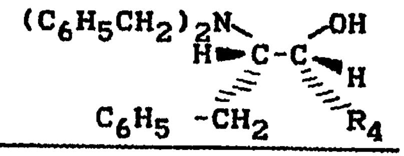 |
|---|---|---|---|---|---|
| $CH_3MgJ$ | 85 | $-CH_3$ | 8 | : | 92 |
| $CH_3Li$ | 86 | $-CH_3$ | 11 | : | 89 |
| $CH_3Ti(OC_3H_7i)_3$ | 89 | $-CH_3$ | 7 | : | 93 |
| $C_6H_5MgBr$ | 84 | $-C_6H_5$ | 3 | : | 97 |
| $CH_2=CHCH_2MgCl$ | 82 | $-CH_2CH=CH_2$ | 28 | : | 72 |
| $CH_2=CHCH_2Ti(OC_3H_7i)_3$ | 84 | $-CH_2CH=CH_2$ | 12 | : | 88 |
| $CH_2=CHCH_2Ti[N(C_2H_5)_2]_3$ | 81 | $-CH_2CH=CH_2$ | 7 | : | 93 |
| $(CH_3)_2Zn/TiCl_4$ | 63 | $-CH_3$ | 78 | : | 22 |
| $CH_2=CHCH_2Si(CH_3)_3/SnCl_4$ | 79 | $-CH_2CH=CH_2$ | 87 | : | 13 |
| $C_6H_5CH_2CH_2MgCl$ | 84 | $-CH_2CH_2C_6H_5$ | 3 | : | 97 |
| $C_6H_5\equiv CLi$ | 72 | $-C\equiv CC_6H_5$ | 2 | : | 98 |
| $(CH_3)_3SiCN/BF_3$ | 74 | CN | 5 | : | 95 |
| $(CH_3)_3SiCN/ZnBr_2$ | 79 | CN | 5 | : | 95 |
| $(CH_3)_3SiCN/MgBr_2$ | 76 | CN | 78 | : | 22 |

Tabelle 3

| Agens | Ausbeute [% d.Th.] | $R_4$ | 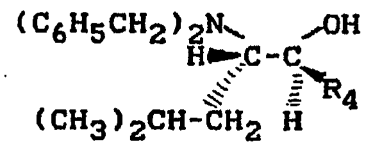 | + | 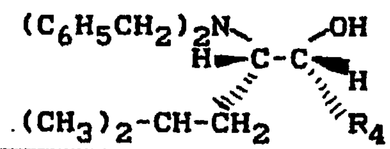 |
|---|---|---|---|---|---|
| $CH_5MgI$ | 85 | $-CH_3$ | 10 | : | 90 |
| $CH_3Li$ | 89 | $-CH_3$ | 20 | : | 80 |
| $CH_3Ti(OC_3H_7i)_3$ | 80 | $-CH_3$ | 6 | : | 94 |
| $C_6H_5MgBr$ | 84 | $-C_6H_5$ | 3 | : | 97 |
| $CH_2=CHCH_2Si(CH_3)_3/SnCl_4$ | 78 | $-CH_2CH=CH_2$ | 90 | : | 10 |
| 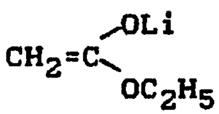 | 75 | $-CH_2COOC_2H_5$ | 10 | : | 90 |
| 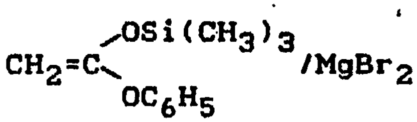 | 60 | $-CH_2COOC_6H_5$ | 88 | : | 12 |

Tabelle 4

| Agens | Ausbeute [% d.Th.] | $R_4$ | 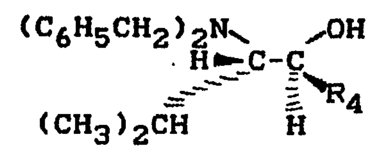 | + | 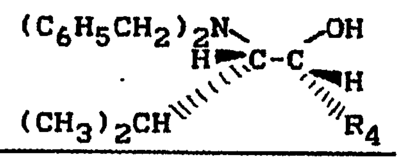 |
|---|---|---|---|---|---|
| $CH_3MgI$ | 87 | $-CH_3$ | 5 | : | 95 |
| $C_6H_5MgBr$ | 69 | $-C_6H_5$ | 9 | : | 91 |
| $CH_2=CHCH_2Ti[N(C_2H_5)_2]_3$ | 83 | $-CH_2CH=CH_2$ | 4 | : | 96 |
| $CH_2=CHCH_2SI(CH_3)_3/SnCl_4$ | 78 | $-CH_2CH=CH_2$ | 95 | : | 5 |

Beispiel 2

a)1,0 g (= 6,1 mmol) Phenylalanin, 3,6 g (= 24 mmol) $K_2CO_3$ in 20 ml $H_2O$ und 2,4 g (= 20 mmol) Allylbromid werden 0,5 Stunden unter Rückfluß gerührt. Anschließend wird das Reaktionsgemisch abgekühlt und mit Essigester extrahiert. Die organische Phase wird mit einer Kochsalzlösung gewaschen und anschließend am Rotationsverdampfer eingeengt. Der Rückstand, gemäß Dünnschichtchromatographie handelt es sich um ein einheitliches Produkt, wird durch Chromatographie an einer Kieselgelsäule (PE/EtOAc; 98:2) gereinigt. Es werden 1,2 g (= 70 % der Theorie) S-2-(N,N-Diallylamino)-β-phenylpropionsäureallylester in Form eines farblosen Öls erhalten.

b)Die Suspension von 0,23 g (= 5,93 mmol) Lithiumaluminiumhydrid in 10 ml Ether wird unter Rühren und Kühlen bei 0°C tropfenweise mit einer Lösung von 1,31 g (= 4,56 mmol) S-2-(N,N-Diallylamino)-3-phenylpropionsäureallylester in 5 ml Ether versetzt. Nach beendeter Zugabe wird das Kühlbad entfernt und das Reaktionsgemisch 2 Stunden bei Raumtemperatur gerührt. Anschließend wird das Reaktionsgemisch durch das Nacheinanderzugeben von 0,23 ml $H_2O$, 0,23 ml 15 %iger Natronlauge und 0,69 ml $H_2O$ hydrolysiert. Die entstandenen Hydroxide werden abfiltriert, zweimal mit Ether ausgekocht, mit wäßriger Schwefelsäure behandelt und erneut mit Ether extrahiert. Die vereinigten Extrakte werden mit $NaCl/H_2O$ gewaschen, über Magnesiumsulfat getrocknet und im Vakuum eingeengt. Der Rückstand wird durch Chromatographie an einer Kieselgel-Säule gereinigt (PE/EtOAc; 95:5). Es werden 0,64 g (= 61 % der Theorie) S-2-(N,N-Diallylamino)-3-phenylpropanol in Form eines farblosen Öls erhalten.

c)Die auf -60°C abgekühlte Lösung von 0,24 g (= 1,8 mmol) Oxalylchlorid in 20 ml Methylenchlorid wird tropfenweise mit 236 mg (= 3,0 mmol) Dimethylsulfoxid versetzt. Die Lösung wird 5 Minuten gerührt und anschließend tropfenweise mit 246 mg (= 0,75 mmol) S-2-(N,N-Diallylamino)-3-phenylpropanol in 3 ml Methylenchlorid versetzt. Nach 45 Minuten werden 610 mg (6,0 mmol) Triethylamin zugegeben. Das Gemisch wird 40 Minuten gerührt und dann zunächst mit $H_2O$ behandelt und anschließend mit 20 ml Methylenchlorid extrahiert. Die organische Phase wird abgetrennt, mit $H_2O$ gewaschen und über $MgSO_4$ getrocknet. Nach dem Entfernen des Lösungsmittels werden 297 mg (= 85 % der Theorid) S-2-(N,N-Diallylamino)-3-phenylpropanal in Form eines farblosen Öls erhalten.

d)Die Lösung von 300 mg (= 1,3 mmol) S-2-(N,N-Diallylamino)-3-phenylpropanal in 1 ml Ether wird zu einer Lösung von 1,4 mmol Methyllithium in 20 ml Ether bei -10°C getropft. Nach 1-stündigem Rühren wird das Reaktionsgemisch durch Zugabe von 4 ml gesättigter Ammoniumchloridlösung hydrolysiert; die organische Phase wird abgetrennt und die wäßrige Phase mit Ether extrahiert. Die vereinigten organischen Phasen werden über $Na_2SO_4$ getrocknet und abschließend im Vakuum vom Lösungsmittel befreit. Der Rückstand wird durch Chromatographie an einer Kieselgelsäule (PE/EtOAc; 96:4) gereinigt. Man erhält 260 mg (= 81 % der Theorie) (2S,3R)-2-(N,N-Diallylamino)-1-phenyl-butanol-3 als Öl. Die $^{13}C$-NMR-spektroskopische Bestimmung der Diastereoselektivität ergab ein Diastereomerenverhältnis von 87:13.

Beispiel 3

Die Suspension von 350 mg (= 1,06 mmol) des in Beispiel 1 bβ) beschriebenen S-2-(N,N-Dibenzylamino)-3-phenylpropionaldehyds und 271 mg (= 1,2 mmol) Zinkbromid in 10 ml Methylenchlorid wird bei -20°C unter Rühren mit 121 mg (= 1,21 mmol) Trimethylsilylcyanid versetzt. Nach 3-stündigem Rühren wird das Reaktionsgemisch mit 5 ml $H_2O$ behandelt. Die organische Phase wird abgetrennt und die wäßrige Phase mit Ether extrahiert. Die vereinigten organischen Phasen werden mit einer Kochsalzlösung gewaschen und über $MgSO_4$ getrocknet. Das nach Entfernen des Lösungsmittels anfallende O-silylierte Cyanhydrin wird durch Behandeln mit 10 %iger wäßriger Lösung von Zitronensäure desilyliert. Das Rohprodukt wird chromatographisch gereinigt (Kieselgel; PE/EtOAc; 50:50). Man erhält 283 mg (= 75 % der Theorie) (2S,3S)-3-(N,N-Dibenzylamino)-2-hydroxy-4-phenyl-butannitril.

Wird als Lewis-Säure statt des $ZnBr_2$ $MgBr_2$ verwendet, so werden 220 mg (= 61 % der Theorie) (2R,3S)-3-(N,N-Dibenzylamino)-2-hydroxy-4-phenylbutannitril erhalten.

Beispiel 4

Zur Suspension von 206 mg (= 1,2 mmol) $MgBr_2$ in 10 ml Methylenchlorid wird bei -15°C unter Rühren 300 mg (= 1,02 mmol) des in Beispiel 1 bβ) beschriebenen S-2-(N,N-Dibenzylamino)-4-methyl-pentanals zugegeben. Nach 20 Minuten werden 253 mg (= 1,22 mmol) 1-Phenoxy-1-trimethylsiloxyethylen zugetropft; die Mischung wird 12 Stunden gerührt und dann auf 5 ml $H_2O$ gegeben. Das Gemisch wird mit einer Mischung von wäßrigem HF/THF behandelt und mehrmals mit Ether extrahiert. Die vereinigten organischen Phasen werden mit $H_2O$ gewaschen, über $MgSO_4$ getrocknet und im Vakuum eingeengt. Das als Rückstand verbleibende Öl wird chromatographisch gereinigt. Es werden 180 mg (= 62 % der Theorie) 4-(N,N-Dibenzylamino)-3-hydroxy-6-methylheptancarbonsäurephenylester erhalten (Diastereomerenverhältnis 3S,4S:3R,4S = 78:22).

## Patentansprüche

1. Optisch aktive α-Aminoaldehyde der Formeln

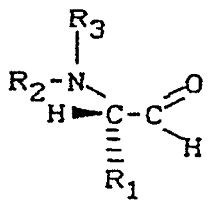

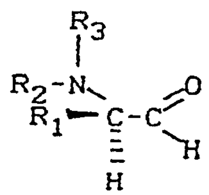

I

in denen
$R_1$ für einen gegebenenfalls substituierten Alkyl-, Alkenyl-, Aralkyl- oder Arylrest steht und
$R_2$ und $R_3$ unabhängig voneinander eine gegebenenfalls substituierte Benzylgruppe bedeuten, zusammen einen gegebenenfalls substituierten Phenylen(1,2)-bis-methylen-Rest bilden oder $R_2$ ein gegebenenfalls substituierter Benzylrest ist und $R_3$ zusamnlen mit $R_1$ einen Propylen-1,3-Rest bildet.

2. Verfahren zur Herstellung von optisch aktiven α-Aminoaldehyden der Formeln

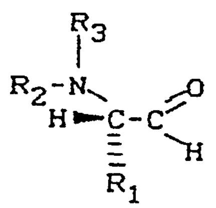

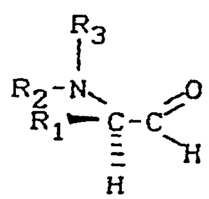

I

in denen
$R_1$ für einen gegebenenfalls substituierten Alkyl-, Alkenyl-, Aralkyl- oder Arylrest steht und
$R_2$ und $R_3$ unabhängig voneinander eine gegebenenfalls substituierte Benzyl-Gruppe bedeuten, zusammen einen gegebenenfalls substituierten Phenylen(1,2)-bis-methylen-Rest bilden oder $R_2$ ein gegebenenfalls substituierter Benzylrest ist und $R_3$ zusammen mit $R_1$ einen Propylen-1,3-Rest bildet, dadurch gekennzeichnet, daß man optisch aktive α-Aminosäuren oder deren Ester der Formel

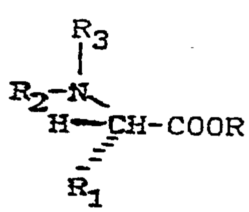

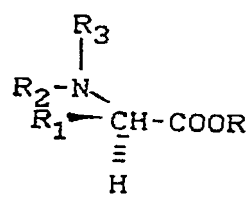

II

in denen
$R_1$, $R_2$ und $R_3$ die unter Formel I angegebene Bedeutung haben und
R für Wasserstoff, einen Alkyl- oder Aralkyl-Rest steht,
nach an sich bekannten Verfahren entweder unmittelbar oder auf dem Umweg über die entsprechenden β-Aminoalkohole zu den α-Aminoaldehyden der Formel I reduziert.

3. Verfahren nach Anspruch 2, dadurch gekennzeichnet, daß man als optisch aktive α-Aminosäuren die in der Natur vorkommenden optisch aktiven α-Aminosäuren verwendet.

4. Verwendung der optisch aktiven α-Aminoaldehyde gemäss Anspruch 1 zur Herstellung von optisch aktiven β-Aminoalkoholen der Formeln

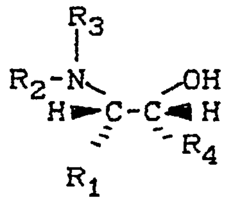

IIIA

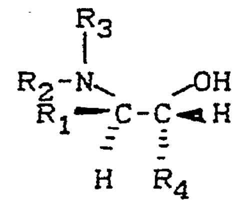

IIIB

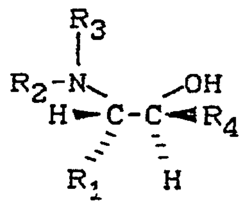

IIIC

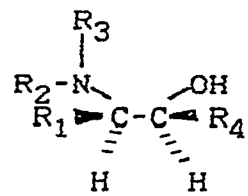

IIID

in denen
$R_1$, $R_2$ und $R_3$ die in Anspruch 1 unter Formel I angegebene Bedeutung haben und
$R_4$ für einen gegebenenfalls substituierten Alkyl-, Alkenyl-, Alkinyl-, Aralkyl-, Aryl- oder Heteroaryl-Rest oder eine CN-Gruppe steht.

**Revendications**

1. α-Aminoaldéhydes, possédant l'activité optique, de formules

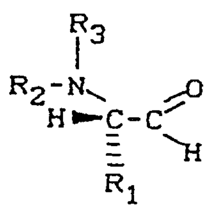

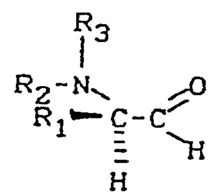

I

dans lesquelles
$R_1$ représente un groupe alkyle, alcényle, aralkyle ou aryle éventuellement substitué et
$R_2$ et $R_3$ représentent chacun, indépendamment l'un de l'autre, un groupe benzyle éventuellement substitué ou forment ensemble un groupe phénylène-(1,2)-bis-méthylène éventuellement substitue, ou bien $R_2$ représente un groupe benzyle éventuellemént substitué et $R_3$ forme avec $R_1$ un groupe propylène-1,3.

2. Procédé de préparation des α-aminoaldéhydes, possédant l'activité optique, de formules

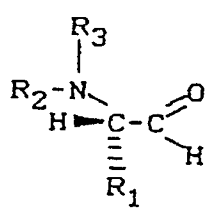

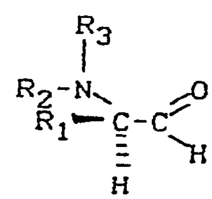

I

dans lesquelles
$R_1$ représente un groupe alkyle, alcenyle, aralkyle ou aryle éventuellement substitué et
$R_2$ et $R_3$ représentent chacun, independamment l'un de l'autre, un groupe benzyle éventuellement substitué ou forment ensemble un groupe phénylène-(1,2)-bis-méthylène éventuellement substitué, ou bien $R_2$ représente un groupe benzyle éventuellement substitué et $R_3$ fcrme avec $R_1$ un groupe propylène-1,3, caractérisé en ce que l'on réduit des α-aminoacides possédant l'activité optique ou leurs esters de formules

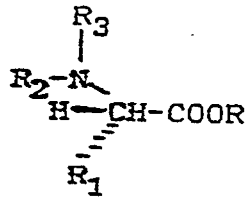

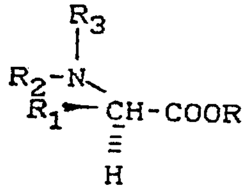

II

dans lesquelles $R_1$, $R_2$ et $R_3$ ont les significations indiquées en référence à la formule I et R représente l'hydrogène, un groupe alkyle ou aralkyle, selon des procédés connus en soi, soit directement, soit en passant par les β-aminoalcools correspondants, en les α-aminoaldéhydes de formule I.

3. Procédé selon la revendication 2, caractérisé en ce que l'on utilise en tant qu'α-aminoacides possédant l'activité optique ceux qui existent dans la nature.

4. Utilisation des α-aminoaldéhydes possédant l'activité optique de la revendication 1 pour la préparation de β-aminoalcools possédant l'activité optique de formules

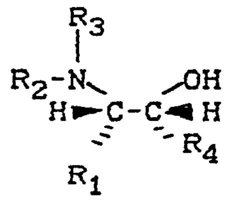

IIIA

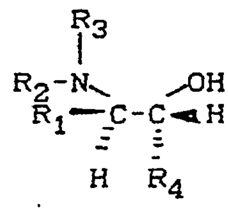

IIIB

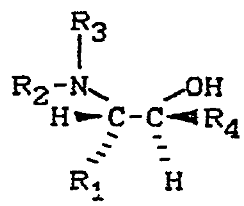

IIIC

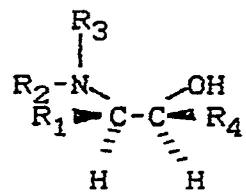

IIID

dans lesquelles
$R_1$, $R_2$ et $R_3$ ont les significations indiquées dans la revendication 1 en référence à la formule I et $R_4$ représente un groupe alkyle, alcényle, alcynyle, aralkyle, aryle ou hétéroaryle éventuellement substitue ou un groupe CN.

## Claims

1. Optically active α-amino aldehydes of the formulae

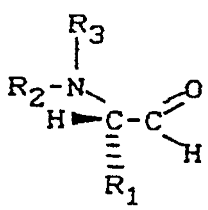

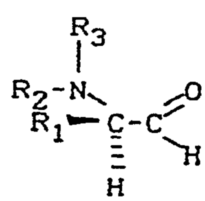

I

in which
$R_1$ represents an optionally substituted alkyl, alkenyl, aralkyl or aryl radical, and $R_2$ and $R_3$, independently of one another, denote an optionally substituted benzyl group, together form an optionally substituted phenylene-(1,2)-bismethylene radical, or $R_2$ is an optionally substituted benzyl radical, and $R_3$ forms together with $R_1$ a 1,3-propylene radical.

2. Process for the preparation of optically active α-amino aldehydes of the formulae

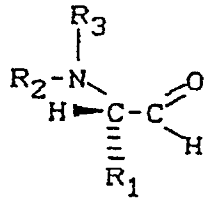 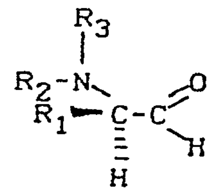 I

in which
$R_1$ represents an optionally substituted alkyl, alkenyl, aralkyl or aryl radical, and $R_2$ and $R_3$, independently of one another, denote an ally substituted benzyl group, together from an optionally substituted phenylene-(1,2)-bismethylene radical, or $R_2$ is an optionally substituted benzyl radical, and $R_3$ forms together with $R_1$ a 1,3-propylene radical, characterized in that optically active α-amino acids or their esters of the formula

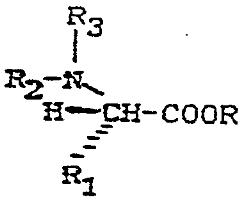 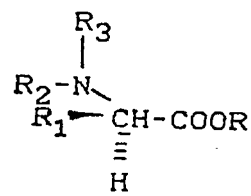 II

in which
$R_1$, $R_2$ and $R_3$ have the meaning indicated under formula I, and
R represents hydrogen, an alkyl or aralkyl radical, are reduced by processes known per se, either directly or indirectly via the corresponding β-amino alcohols, to give the α-amino aldehydes of the formula I.

3. Process according to Claim 2, characterized in that naturally occurring optically active α-amino acids are used as optically active α-amino acids.

4. Use of the optically active α-amino aldehydes according to Claim 1 for the preparation of optically active β-amino alcohols of the formulae

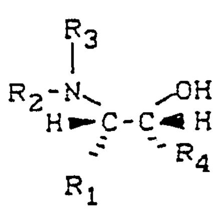

IIIA

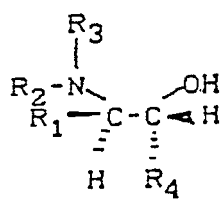

IIIB

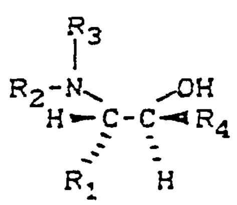

IIIC

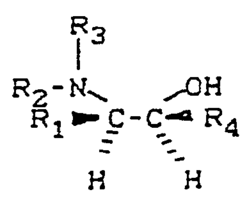

IIID

in which
$R_1$, $R_2$ and $R_3$ have the meaning indicated under formula I in Claim 1, and $R_4$ represents an optionally substituted alkyl, alkenyl, alkynyl, aralkyl, aryl or heteroaryl radical, or a CN group.